# EUROPÄISCHE PATENTSCHRIFT

(11) **EP 0 893 427 B1**
(45) Veröffentlichungstag und Bekanntmachung des Hinweises auf die Patenterteilung: **19.09.2001**
(21) Anmeldenummer: 98112830.9
(22) Anmeldetag: 10.07.1998
(51) Int. Cl.: C07C 67/347, C07C 69/757

(54) **Verfahren zur Herstellung von Cyclobutan-1,2-dicarbonsäureestern**
Process for the preparation of cyclobutan-1,2-dicarboxylic acid esters
Procédé de préparation d'esters de l'acide cyclobutane-1,2-dicarboxylique

(30) Priorität: 22.07.1997 CH 177297
(43) Veröffentlichungstag der Anmeldung: 27.01.1999
(73) Patentinhaber: LONZA A.G., CH-4002 Basel (CH)
(72) Erfinder: Brunner, Andreas, 3900 Brig (CH)

(56) Entgegenhaltungen:
- EP-A- 0 458 643
- SALEEM AHMAD: "An Efficient and Diastereoselective (2+2) Cycloaddition:Convenient and Enantioselective Route to Trans-2',3'-Dihydroxymethylcyclobutane Nucleoside Analogs" TETRAHEDRON LETTERS., Bd. 32, Nr. 48, 25. November 1991, Seiten 6997-7000, XP002080843 OXFORD GB
- KEIJI MARUOKA ET AL.: "Regiocontrolled (2+2) Cycloaddition of Unsymmetrical Fumarates Based on the Discrimination of Two Different Ester Carbonyls with Methylaluminium Bis(2,6-di-tert-butyl-4-methylphenoxide) (MAD)" SYNLETT., Nr. 3, März 1993, Seiten 197-198, XP002080844 STUTTGART DE

## Beschreibung

Die vorliegende Erfindung betrifft ein Verfahren zur Herstellung von Cyclobutan-1,2-dicarbonsäureestern (I) durch [2 + 2]-Cycloaddition von Ketenacetalen und Fumar- oder Maleinsäureestern.

Die erfindungsgemäss herstellbaren Verbindungen besitzen die allgemeine Formel

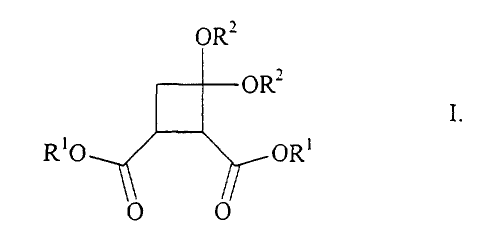

Darin bedeutet R¹ eine C₁₋₈-Alkylgruppe, eine gegebenenfalls substituierte mono- oder bicyclische cycloaliphatische Gruppe mit 3 bis 10 Ringkohlenstoffatomen, eine gegebenenfalls substituierte Aryl- oder Arylalkylgruppe oder eine gegebenenfalls substituierte gesättigte heterocyclische Gruppe und R² ist C₁₋₄-Alkyl oder beide Reste R² zusammen sind ―(CH₂)_{*n*}― mit *n* = bis 4.
Unter C₁₋₄- bzw. C₁₋₈-Alkylgruppen sind hier und im folgenden jeweils alle linearen oder verzweigten primären, sekundären oder tertiären Alkylgruppen mit 1 bis 4 bzw. 1 bis 8 Kohlenstoffatomen zu verstehen, also unter C₁₋₄-Alkyl Methyl, Ethyl, Propyl, Isopropyl, Butyl, Isobutyl, *sec*-Butyl und *tert*-Butyl und unter C₁₋₈-Alkyl zusätzlich beispielsweise noch Pentyl, Isopentyl, Neopentyl, Hexyl oder Octyl. Entsprechend sind unter C₁₋₄-Alkoxy die aus den vorstehend definierten C₁₋₄-Alkylgruppen und Sauerstoff zusammengesetzten Gruppen zu verstehen. Unter mono- oder bicyclischen cycloaliphatischen Gruppen mit 3 bis 10 Kohlenstoffatomen sind beispielsweise Gruppen wie Cyclopropyl, Cyclobutyl, Cyclopentyl, Cyclohexyl, Cycloheptyl, Norboman-x-yl, Norcaran-x-yl oder Norpinan-x-yl zu verstehen, wobei das "x" für eine beliebige Verknüpfungsposition steht. Diese Gruppen können auch einen oder mehrere Substituenten tragen, beispielsweise C₁₋₄-Alkylgruppen. Zu diesen substituierten cycloaliphatischen Gruppen zählen beispielsweise Menthan-x-yl, Bornan-x-yl, Caran-x-yl, Pinan-x-yl oder Thujan-x-yl. Unter Arylgruppen sind aromatische Kohlenwasserstoffreste mit einem oder mehreren Ringen zu verstehen, also beispielsweise Phenyl, x-Naphthyl, Anthracen-x-yl, Phenanthren-x-yl, Fluoren-x-yl oder Biphenyl-x-yl. Bei Resten mit mehreren Ringen können diese auch teilweise hydriert sein wie beispielsweise Tetrahydronaphthalin-x-yl, Indan-x-yl oder Acenaphthen-x-yl. Beispiele für Arylalkylgruppen sind Benzyl, 1-Phenylethyl, 2-Phenylethyl (Phenethyl) oder Diphenylmethyl (Benzhydryl). Gesättigte heterocyclische Gruppen sind beispielsweise Tetrahydrofuryl oder Tetrahydropyranyl. Diese Aryl-, Arylalkyl- oder gesättigten heterocyclischen Gruppen können gegebenenfalls einen oder mehrere gleiche oder verschiedene Substituenten wie beispielsweise C₁₋₄-Alkylgruppen, C₁₋₄-Alkoxygruppen oder Halogenatome tragen.

Die erfindungsgemäss herstellbaren Verbindungen, insbesondere solche mit *trans*-Konfiguration am Cyclobutanring, sind Zwischenprodukte in der Synthese von pharmazeutischen Wirkstoffen, beispielsweise von antiviralen Nucleosidanalogen (EP-A-0 458 643).

Es ist bekannt, dass 3,3-Dialkoxycyclobutan-1,2-dicarbonsäureester durch [2 + 2]-Cycloaddition aus Fumar- oder Maleinsäureestern und Ketenacetalen hergestellt werden können. Da die Cycloaddition stereospezifisch verläuft, werden aus Fumarsäureestern Produkte mit *trans*-Konfiguration am Cyclobutanring erhalten, und zwar normalerweise in Form eines Racemats. Es ist weiterhin bekannt, dass bei Verwendung von Estern der Fumarsäure mit optisch aktiven Alkoholen Diastereomerengemische erhalten werden, in denen eines der beiden Stereoisomeren (je nach Art und Konfiguration der Alkoholkomponente dasjenige mit (1*S*)- oder (1*R*)-Konfiguration am Cyclobutanring) vorherrscht (EP-A-0 458 643, Example 1; S. Ahmad, *Tetrahedron Lett.* **1991,** *32,* 6997-7000). Das Verfahren erfordert den Einsatz von wenigstens 2 Äquivalenten Dialkylaluminiumchlorid. Ein weiterer Nachteil dieses Verfahrens liegt darin, dass es bei tiefen Temperaturen von beispielsweise -75 °C durchgeführt werden muss, um eine gute Ausbeute zu erreichen. Derart tiefe Temperaturen sind jedoch in technischem Massstab nur mit erheblichem Aufwand zu erreichen.

Aufgabe der vorliegenden Erfindung war daher, ein Verfahren bereitzustellen, welches keinen grossen Überschuss an teurem Dialkylaluminiumchlorid benötigt und bei weniger tiefen Temperaturen durchgeführt werden kann.

Erfindungsgemäss wird diese Aufgabe durch das Verfahren nach Patentanspruch 1 gelöst.

Es wurde überraschend gefunden, dass bei der Umsetzung von Dicarbonsäureestern der allgemeinen Formel 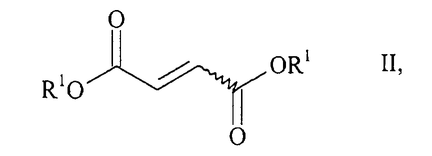 worin R¹ die oben genannte Bedeutung hat, mit einem Ketenacetal der allgemeinen Formel 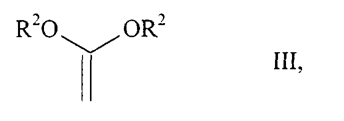 in Gegenwart einer Lewis-Säure durch Zusatz einer sterisch gehinderten Base zu dem Reaktionsgemisch nicht nur eine verbesserte Ausbeute erzielt werden kann, sondern auch eine Durchführung der Reaktion mit geringerem Überschuss an Lewis-Säure (z. B. Dialkylaluminiumchlorid) und bei höherer Temperatur (bis ca. +20 °C und darüber) möglich ist.

In einer bevorzugten Ausführungsform des erfindungsgemässen Verfahrens wird als Dicarbonsäureester (II) ein Fumarsäureester der allgemeinen Formel 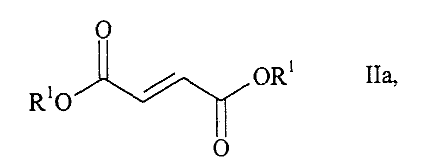 worin R¹ die oben genannte Bedeutung hat, eingesetzt und ein Cyclobutan-*trans*-1,2-di-carbonsäureester der allgemeinen Formel 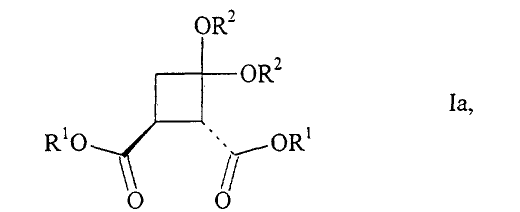 und/oder Spiegelbild,
worin R¹ und R² die oben genannten Bedeutungen haben, erhalten.

In einer anderen Ausführungsform wird als Dicarbonsäureester (II) ein Maleinsäureester der allgemeinen Formel 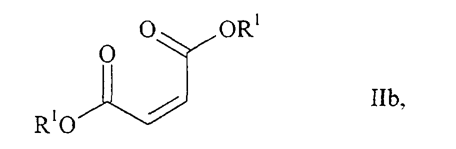 worin R¹ die oben genannte Bedeutung hat, eingesetzt und ein Cyclobutan-*cis*-1,2-di-carbonsäureester der allgemeinen Formel 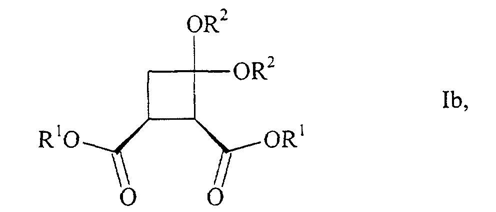 und/oder Spiegelbild,
worin R¹ und R² die oben genannten Bedeutungen haben, erhalten.

Vorzugsweise werden solche Dicarbonsäureester (II) eingesetzt, in denen R¹ wenigstens ein Chiralitätselement enthält, und zwar in nicht-racemischer Form. Besonders bevorzugt sind diese nicht-racemischen Dicarbonsäureester (II) im wesentlichen optisch rein. Die aus chiralen Dicarbonsäureestern (II) entstehenden Cyclobutan-1,2-dicarbonsäureester-Stereoisomeren (I) sind keine Enantiomeren, sondern Diastereomere, die sich in ihren physikalischen Eigenschaften unterscheiden und mehr oder weniger leicht trennen lassen. Die [2 + 2]-Cycloaddition läuft mit diesen Dicarbonsäureestern (II) stereoselektiv in Bezug auf die absolute Konfiguration am entstehenden Cyclobutanring ab, so dass ein Diastereomer bevorzugt gebildet wird.

Als chirale Reste R¹ werden vorzugsweise Reste wie 1-Phenylethyl, Menthyl und dessen Stereoisomere, Bornyl und dessen Stereoisomere, Dihydro-4,4-dimethylfuran-2(3*H*)-on-3-yl (abgeleitet von Pantolacton), 1-(Methoxycarbonyl)ethyl oder 1-(Ethoxycarbonyl)ethyl (abgeleitet von Methyl- bzw. Ethyllactat) eingesetzt. Besonders bevorzugt ist Menthyl. *Mit* optisch reinen Fumarsäuredimenthylestern lassen sich sehr gute Diastereoselektivitäten erzielen und es können Cyclobutan-*trans*-1,2-dicarbonsäureester (Ia) mit *de*-Werten (*de =* diastereomeric excess, definiert als Quotient |A - B| : (A + B), worin A und B für die Mengen der beiden Diastereomeren stehen) von bis zu 90% und mehr erhalten werden, die nach einem einfachen Reinigungsschritt (z. B. Umkristallisation) praktisch stereoisomerenrein sind.

Insbesondere werden aus Fumarsäuredi-(1*R*)-menthylestern (IIa, R¹ = (1*R*)-Menthyl) vorwiegend Cyclobutan-*trans*-1,2-dicarbonsäureester (Ia) in der (1*S*)-Konfiguration des Cyclobutanrings erhalten.

Als Ketenacetale (III) werden vorzugsweise solche eingesetzt, in denen R² Methyl oder Ethyl ist oder beide Reste R² zusammen ―(CH₂)₄― sind. Namentlich sind dies Ketendimethylacetal, Ketendiethylacetal und 2-Methylen-1,3-dioxepan.

Als Lewis-Säuren eignen sich beispielsweise Organoaluminium- und Organotitanverbindungen der allgemeinen Formeln R₂AlCl, RAlCl₂, Al(OR)₃, Ti(OR)₂Cl₂, Ti(OR)₄, worin die Reste R gleich oder verschieden sind und jeweils für eine C₁₋₈-Alkylgruppe stehen, sowie Halogenide wie AlCl₃, TiCl₄, BF₃, ZnCl₂, ZnBr₂, ZrCl₄ und SnCl₄. Vorzugsweise wird als Lewis-Säure ein Dialkylaluminiumchlorid der allgemeinen Formel R₂AlCl eingesetzt. Besonders bevorzugt sind die Dialkylaluminiumchloride, in denen R Ethyl oder Isobutyl ist.

Als sterisch gehinderte Basen eignen sich insbesondere tertiäre Amine wie beispielsweise 2,6-Di*-tert*-butylpyridin, 2,6-Di*-tert*-butylpyridin an polymeren Träger gebunden, 2,6-Di-*tert*-butyl-4-methylpyridin, Triethylamin, Diethylisopropylamin, Ethyldiisopropylamin, Triisopropylamin und *N*-Ethyl-dicyclohexylamin. Besonders bevorzugt ist Ethyldiisopropylamin ("Hünigs Base").

Die nachfolgenden Beispiele verdeutlichen die Durchführung des erfindungsgemässen Verfahrens, ohne dass darin eine Einschränkung zu sehen ist.

### Beispiele

### Beispiel 1

### (1S,2R)-3,3-Diethoxy-1,2-cyclobutandicarbonsäuredi-(1R)-menthylester

In einem Doppelmantelkolben mit Kühlung durch einen Umlaufkryostaten wurden bei Raumtemperatur unter Argon 30,0 g (76,5 mmol) Fumarsäuredi-(1*R*)-menthylester in 250 ml Toluol gelöst. Die Lösung wurde auf-21 °C abgekühlt und innerhalb von 15 min mit 16,5 ml (84,5 mmol) Diisobutylaluminiumchlorid versetzt. Nach weiteren 5 min wurden 5,2 ml (30,4 mmol) Ethyldiisopropylamin zugegeben. Das Gemisch wurde noch weitere 5 min gerührt und dann innerhalb von 15 min unter verstärkter Kühlung (Kühlmitteltemperatur: -24 °C) mit 11,1 ml (84,1 mmol) Ketendiethylacetal versetzt, wobei die Innentemperatur auf-19 °C anstieg. Das dunkle Reaktionsgemisch wurde noch 40 min bei -22 bis -20 °C gerührt und danach auf ein Gemisch von 200 ml *n*-Hexan, 200 ml gesättigter wässriger Natriumhydrogencarbonatlösung und wenig Eis gegossen. Die entstandene orange Emulsion wurde bis zum Erreichen der Raumtemperatur gerührt. Anschliessend wurden die Phasen getrennt und die wässrige Phase mit 2 × 200 ml Hexan extrahiert. Die vereinigten organischen Phasen wurden je zweimal mit gesättigter Natriumhydrogencarbonatlösung und gesättigter Kochsalzlösung gewaschen, über Celite® filtriert und über Natriumsulfat getrocknet. Das Lösungsmittel wurde im Vakuum abdestilliert und der Rückstand im Hochvakuum getrocknet. Das so erhaltene Rohprodukt (40,38 g, oranger Feststoff) enthielt nach NMR kein unumgesetztes Ausgangsmaterial und bestand nach GC aus einem Gemisch der (1*S*,2*R*)- und (1*R*,2*S*)-Diastereomeren im Verhältnis 93,5:6,5 (87% *de*). Zur Reinigung wurden 39,1 g des Rohprodukts in 1,2 l Methanol/Wasser (95:5) heiss gelöst, unter Rühren auf Raumtemperatur abgekühlt und über Nacht bei 0 °C stehengelassen. Die ausgefallenen gelblichen Kristalle wurden abfiltriert, mit wenig Methanol/Wasser gewaschen und im Hochvakuum getrocknet.
Ausbeute: 27,9 g (74%, bezogen auf Fumarsäuredimenthylester), diastereomerenrein.

### Beispiel 2

### (1S,2R)-3,3-Dimethoxy-1,2-cyclobutandicarbonsäuredi-(1R)-menthylester

Analog zu Beispiel 1 wurde Fumarsäuredi-(1*R*)-menthylester in Gegenwart von 0,2 Äquivalenten Ethyldiisopropylamin und 2 Äquivalenten Diethylaluminiumchlorid bei -40°C mit Ketendimethylacetal umgesetzt. Nach ¹H NMR-Analyse des Reaktionsgemisches war die Ausbeute quantitativ und der *de-*Wert >80%.

### Vergleichsbeispiel 1

### (1S,2R)-3,3-Dimethoxy-1,2-cyclobutandicarbonsäuredi-(1R)-menthylester

Es wurde wie in Beispiel 2 verfahren, jedoch ohne Zusatz von Ethyldiisopropylamin. Nach ¹H NMR-Analyse des Reaktionsgemisches war die Ausbeute 37% und der *de*-Wert >80%.

### Beispiel 3

### (1S,2R)-5,10-Dioxaspiro[3.6]decan-1,2-dicarbonsäuredi-(1R)-menthylester [I, R¹ = (1R)-Menthyl, 2 R² = ―(CH₂)₄―]

Analog zu Beispiel 1 wurde Fumarsäuredi-(1*R*)-menthylester in Gegenwart von 0,2 Äquivalenten Ethyldiisopropylamin und 2 Äquivalenten Diethylaluminiumchlorid bei -20 °C mit 2-Methylen-1,3-dioxepan (Ketentetramethylenacetal) umgesetzt. Nach ¹H NMR-Analyse des Reaktionsgemisches war die Ausbeute 93% und es war nur ein Diastereomeres nachweisbar. Eine Wiederholung des Versuchs bei 0 °C Reaktionstemperatur brachte das gleiche Resultat.

### Vergleichsbeispiel 2

### (1S,2R)-5,10-Dioxaspiro[3.6]decan-1,2-dicarbonsäuredi-(1R)-menthylester

Es wurde wie in Beispiel 3 verfahren, jedoch ohne Zusatz von Ethyldiisopropylamin. Bei einer Reaktionstemperatur von -40 °C wurde eine Ausbeute von 73% erzielt, bei 0 °C lediglich 33%.

### Beispiel 4

### (±)-3,3-Diethoxy-trans-1,2-cyclobutandicarbonsäurediethylester

Analog zu Beispiel 1 wurde Fumarsäurediethylester in Gegenwart von 0,2 Äquivalenten Ethyldiisopropylamin und 2 Äquivalenten Diisobutylaluminiumchlorid bei -40°C mit 2 Äquivalenten Ketendiethylacetal umgesetzt. Nach ¹H NMR-Analyse des Reaktionsgemisches war die Ausbeute quantitativ.

### Beispiele 5-14, Vergleichsbeispiele 3-4

### (1S,2R)-3,3-Diethoxy-1,2-cyclobutandicarbonsäuredi-(1R)-menthylester

Es wurde analog zu Beispiel 1 verfahren, wobei jedoch die Mengen von Ketendiethylacetal, Ethyldiisopropyamin und Dialkylaluminiumchlorid sowie Reaktionstemperatur. Lösungsmittel und der Alkylrest im Dialkylaluminiumchlorid variiert wurden. Die Bedingungen und die mit GC bestimmten Ausbeuten und *de*-Werte sind in der folgenden Tabelle 1 zusammengefasst.

**Tabelle 1**

| Bsp. Nr. | Acetal [Äq.] | Amin [Äq.] | Temp. [°C] | R | R₂AlCl [Äq.] | Lsgm. | Ausb. [%] | *de* [%] |
|---|---|---|---|---|---|---|---|---|
| 5 | 1,1 | 0,4 | -20 | Et | 2,0 | Toluol | ≈100 | 86,6 |
| 6 | 1,1 | 0,4 | -20 | ⁱBu | 2,0 | Toluol | ≈100 | 90,7 |
| 7 | 1,1 | 0,4 | 0 | Et | 2,0 | Toluol | 95 | 78,9 |
| 8 | 1,1 | 0,4 | 0 | ⁱBu | 2,0 | Toluol | ≈100 | 84,7 |
| 9 | 1,1 | 0,4 | +20 | ⁱBu | 2,0 | Toluol | ≈100 | 73 |
| 10 | 1,1 | 0,4 | -20 | ⁱBu | 1,1 | Toluol | ≈100 | 90,9 |
| 11 | 1,1 | 0,4 | -40 | ⁱBu | 1,1 | Toluol | ≈100 | 94.8 |
| 12 | 1,1 | 0,4 | -20 | Et | 2,0 | CH₂Cl₂ | ≈100 | 83.2 |
| 13 | 1,1 | 0,4 | -20 | ⁱBu | 2,0 | CH₂Cl₂ | ≈100 | 83.1 |
| 14 | 1,1 | 0,4 | -20 | ⁱBu | 1,1 | C₂H₄Cl₂ | ≈100 | 90.9 |
| V 3 | 1,5 | ― | -78 | Et | 2,0 | Toluol | <10 | n.b. |
| V 4 | 1,5 | ― | -40 | Et | 2,0 | Toluol | 29 | n.b. |

## Patentansprüche

1. Verfahren zur Herstellung von Cyclobutan-1,2-dicarbonsäureestern der allgemeinen Formel 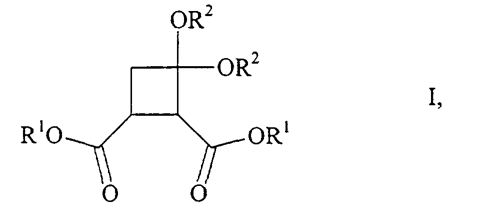 worin R¹ eine C₁₋₈-Alkylgruppe, eine gegebenenfalls substituierte mono- oder bicyclische cycloaliphatische Gruppe mit 3 bis 10 Ringkohlenstoffatomen, eine gegebenenfalls substituierte Aryl- oder Arylalkylgruppe oder eine gegebenenfalls substituierte gesättigte heterocyclische Gruppe ist und R² entweder C₁₋₄-Alkyl bedeutet oder beide Reste R² zusammen eine Gruppe der allgemeinen Formel ―(CH₂)_{*n*}―, worin *n* eine ganze Zahl von 2 bis 4 ist, bilden,
durch Umsetzung eines Dicarbonsäureesters der allgemeinen Formel 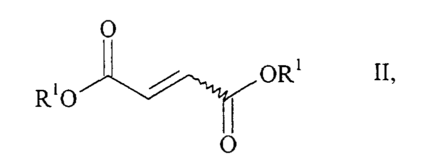 worin R¹ die oben genannte Bedeutung hat, mit einem Ketenacetal der allgemeinen Formel 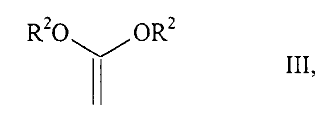 worin R² die oben genannte Bedeutung hat, in Gegenwart einer Lewis-Säure, **dadurch gekennzeichnet, dass** die Reaktion in Gegenwart einer sterisch gehinderten Base durchgeführt wird.

2. Verfahren nach Anspruch 1, **dadurch gekennzeichnet, dass** als Dicarbonsäureester (II) ein Fumarsäureester der allgemeinen Formel 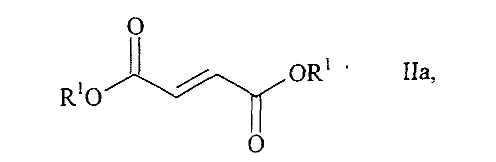 worin R¹ die oben genannte Bedeutung hat, eingesetzt und ein Cyclobutan-*trans*-1,2-dicarbonsäureester der allgemeinen Formel 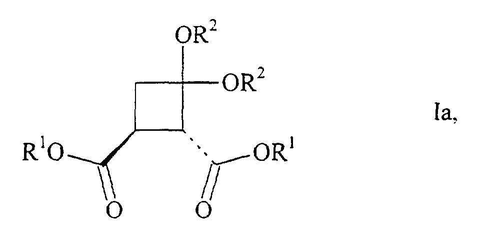 und/oder Spiegelbild,
worin R¹ und R² die oben genannten Bedeutungen haben, erhalten wird.

3. Verfahren nach Anspruch 1, **dadurch gekennzeichnet, dass** als Dicarbonsäureester (II) ein Maleinsäureester der allgemeinen Formel 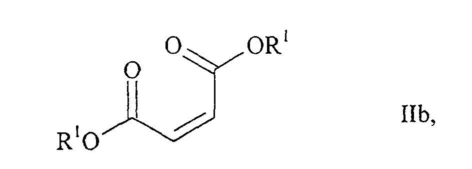 worin R¹ die oben genannte Bedeutung hat, eingesetzt und ein Cyclobutan-*cis*-1,2-dicarbonsäureester der allgemeinen Formel 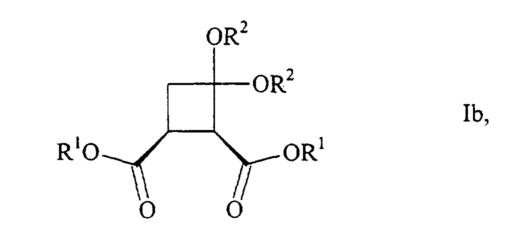 und/oder Spiegelbild,
worin R¹ und R² die oben genannten Bedeutungen haben, erhalten wird.

4. Verfahren nach einem der Ansprüche 1 bis 3, **dadurch gekennzeichnet, dass** R¹ wenigstens ein Chiralitätselement enthält und der Dicarbonsäureester (II) in nicht-racemischer Form vorliegt.

5. Verfahren nach Anspruch 4, **dadurch gekennzeichnet, dass** R¹ ausgewählt ist aus der Gruppe bestehend aus 1-Phenylethyl, Menthyl und Bornyl und deren Stereoisomeren, Dihydro-4,4-dimethylfuran-2(3*H*)-on-3-yl, 1-(Methoxycarbonyl)ethyl und 1-(Ethoxycarbonyl)ethyl.

6. Verfahren nach Ansprüchen 2 und 5, **dadurch gekennzeichnet, dass** R¹ (1*R*)-Menthyl ist und der Cyclobutan*-trans*-1,2-dicarbonsäureester (Ia) vorwiegend in der (1*S*)-Konfiguration erhalten wird.

7. Verfahren nach einem der Ansprüche 1 bis 6, **dadurch gekennzeichnet, dass** R² Methyl oder Ethyl ist oder beide Reste R² zusammen ―(CH₂)₄― sind.

8. Verfahren nach einem der Ansprüche 1 bis 7, **dadurch gekennzeichnet, dass** als Lewis-Säure ein Dialkylaluminiumchlorid der allgemeinen Formel R₂AlCl, worin R eine C₁₋₈-Alkylgruppe ist, eingesetzt wird.

9. Verfahren nach Anspruch 8, **dadurch gekennzeichnet, dass** R Ethyl oder Isobutyl ist.

10. Verfahren nach einem der Ansprüche 1 bis 9, **dadurch gekennzeichnet, dass** als sterisch gehinderte Base Ethyldiisopropylamin eingesetzt wird.

## Claims

1. Process for preparing cyclobutane-1,2-dicarboxylic esters of the general formula 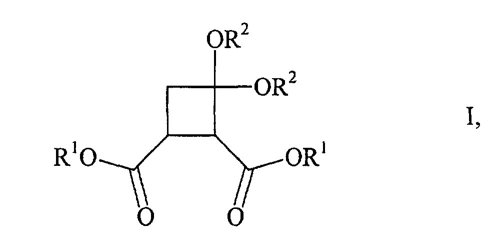 in which R¹ is a C₁₋₈-alkyl group, an optionally substituted mono- or bicyclic cycloaliphatic group having 3 to 10 ring carbon atoms, an optionally substituted aryl or arylalkyl group or an optionally substituted saturated heterocyclic group and R² is either C₁₋₄-alkyl or both radicals R² together form a group of the general formula -(CH₂)_{*n*}- where *n* is an integer from 2 to 4,
by reacting a dicarboxylic ester of the general formula 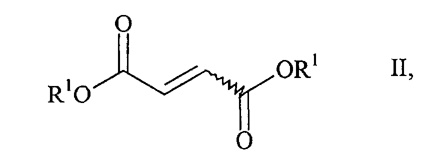 in which R¹ is as defined above, with a ketene acetal of the general formula 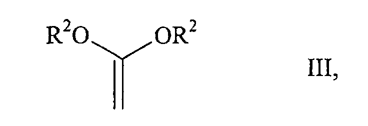 in which R² is as defined above, in the presence of a Lewis acid, **characterized in that** the reaction is carried out in the presence of a sterically hindered base.

2. Process according to Claim 1, **characterized in that** the dicarboxylic ester (II) employed is a fumaric ester of the general formula 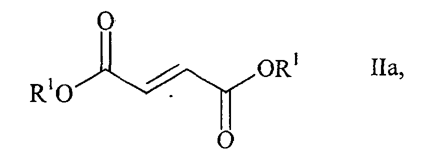 in which R¹ is as defined above, and a cyclobutane-trans-1,2-dicarboxylic ester of the general formula 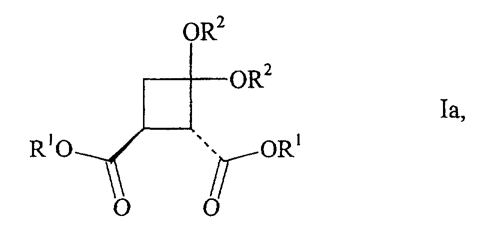 and/or the mirror image,
in which R¹ and R² are as defined above, is obtained.

3. Process according to Claim 1, **characterized in that** the dicarboxylic ester (II) employed is a maleic ester of the general formula 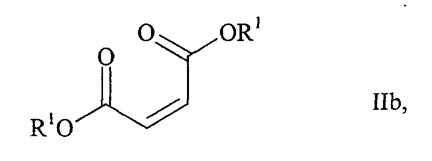 in which R¹ is as defined above, and a cyclobutane-*cis*-1,2-dicarboxylic ester of the general formula 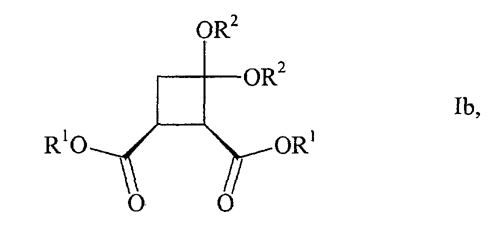 and/or the mirror image,
in which R¹ and R² are as defined above, is obtained.

4. Process according to any of Claims 1 to 3, **characterized in that** R¹ contains at least one chiral element and the dicarboxylic ester (II) is present in nonracemic form.

5. Process according to Claim 4, **characterized in that** R¹ is selected from the group consisting of 1-phenylethyl, menthyl and bornyl and their stereoisomers, dihydro-4,4-dimethylfuran-2(*3H*)-on-3-yl, 1-(methoxycarbonyl)ethyl and 1-(ethoxycarbonyl)ethyl.

6. Process according to Claims 2 and 5, **characterized in that** R¹ is (1*R*)-menthyl and the cyclobutane-trans-1,2-dicarboxylic ester (Ia) is obtained predominantly in the (1*S*) configuration.

7. Process according to any of Claims 1 to 6, **characterized in that** R² is methyl or ethyl or both radicals R² together are -(CH₂)₄-.

8. Process according to any of Claims 1 to 7, **characterized in that** the Lewis acid employed is a dialkylaluminium chloride of the general formula R₂AlCl, in which R is a C₁₋₈-alkyl group.

9. Process according to Claim 8, **characterized in that** R is ethyl or isobutyl.

10. Process according to any of Claims 1 to 9, **characterized in that** the sterically hindered base employed is ethyldiisopropylamine.

## Revendications

1. Procédé pour la préparation d'esters d'acide cyclobutane-1,2-dicarboxylique de formule générale 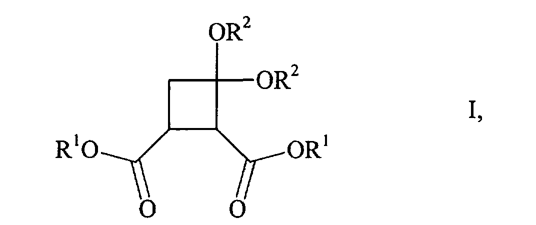 dans laquelle R¹ représente un groupe alkyle en C₁₋₈, un groupe cycloaliphatique mono- ou bicyclique éventuellement substitué, ayant de 3 à 10 atomes de carbone formant le cycle, un groupe aryle ou arylalkyle éventuellement substitué ou un groupe hétérocyclique saturé éventuellement substitué, et soit R² représente un groupe alkyle en C₁₋₄, soit les deux radicaux R² forment ensemble un groupe de formule générale -(CH₂)_{*n*}-, dans laquelle *n* est un nombre entier allant de 2 à 4,
par la réaction d'un ester d'acide dicarboxylique de formule générale 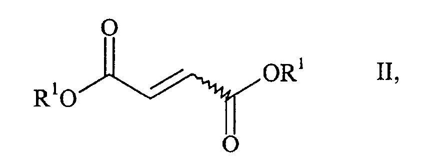 dans laquelle R¹ a la signification donnée ci-dessus, avec un cétènacétal de formule générale 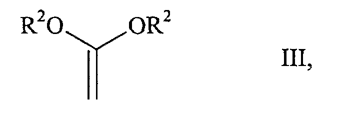 dans laquelle R² a la signification donnée plus haut, en présence d'un acide de Lewis, **caractérisé en ce que** la réaction est effectuée en présence d'une base à empêchement stérique.

2. Procédé selon la revendication 1, **caractérisé en ce que**, comme ester d'acide dicarboxylique (II), on utilise un ester d'acide fumarique de formule générale 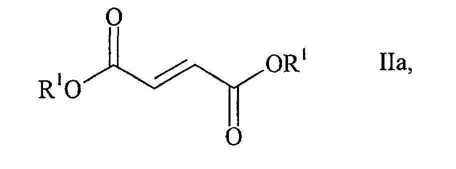 dans laquelle R¹ a la signification donnée plus haut, et on obtient un ester d'acide cyclobutane-*trans-*1,2-dicarboxylique de formule générale 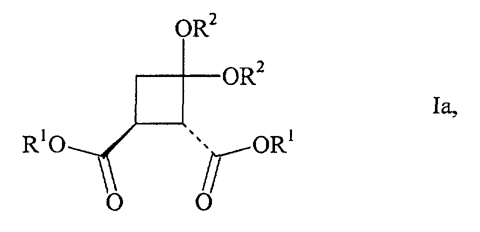 et/ou son image dans un miroir,
formule dans laquelle R¹ et R² ont les significations données plus haut.

3. Procédé selon la revendication 1, **caractérisé en ce que**, comme ester d'acide dicarboxylique (II), on utilise un ester d'acide maléique de formule générale 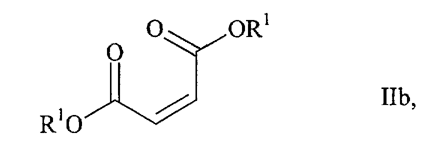 dans laquelle R¹ a la signification donnée plus haut, et on obtient un ester d'acide cyclobutane*-cis*-1,2-dicarboxylique de formule générale 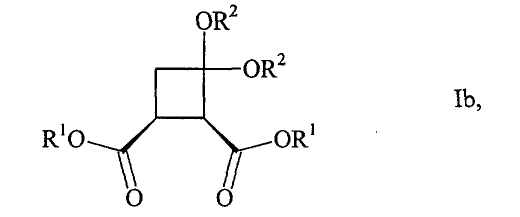 et/ou son image dans un miroir,
formule dans laquelle R¹ et R² ont les significations données plus haut.

4. Procédé selon l'une quelconque des revendications 1 à 3, **caractérisé en ce que** R¹ comporte au moins un élément de chiralité et l'ester d'acide dicarboxylique (II) se trouve sous forme non racémique.

5. Procédé selon la revendication 4, **caractérisé en ce que** R¹ est choisi dans le groupe constitué par les groupes 1-phényléthyle, menthyle et bornyle et leurs stéréoisomères, dihydro-4,4-diméthylfurann-2(3*H*)-on-3-yle, 1-(méthoxycarbonyl)éthyle et 1-(éthoxycarbonyl)éthyle.

6. Procédé selon les revendications 2 et 5, **caractérisé en ce que** R¹ est le groupe (1*R*)-menthyle, et l'ester d'acide cyclobutane-*trans*-1,2-dicarboxylique (Ia) est obtenu principalement en la configuration (1*S*).

7. Procédé selon l'une quelconque des revendications 1 à 6, **caractérisé en ce que** R² est le groupe méthyle ou éthyle, ou les deux radicaux R² forment ensemble -(CH₂)₄-.

8. Procédé selon l'une quelconque des revendications 1 à 7, **caractérisé en ce qu'**on utilise comme acide de Lewis un chlorure de dialkylaluminium de formule générale R₂AlCl, dans laquelle R est un groupe alkyle en C₁₋₈.

9. Procédé selon la revendication 8, **caractérisé en ce que** R est le groupe éthyle ou isobutyle.

10. Procédé selon l'une quelconque des revendications 1 à 9, **caractérisé en ce que**, comme base à empêchement stérique, on utilise l'éthyldiisopropylamine.
